# EUROPEAN PATENT APPLICATION

(11) **EP 1 721 569 A1**
(43) Date of publication of application: **15.11.2006**
(21) Application number: 06113730.3
(22) Date of filing: 09.05.2006
(51) Int. Cl.: A61B 3/06, G09G 5/02, G06F 3/033, H04N 1/60

(54) **Display Apparatus**

(30) Priority: 10.05.2005 KR 2005038943
(71) Applicant: Samsung Electronics Co., Ltd., Suwon-si 442-742 Gyeonggi-Do (KR)
(72) Inventor: Cho, Cheon-yong, Gyeonggi-do (KR); Baek, Joung-hum, Gyeonggi-do (KR); Jang, Hyun-young, Seoul (KR)
(74) Representative: Walaski, Jan Filip

(57) **Abstract**

A method of controlling a display apparatus includes displaying a first graphic user interface to determine whether a user has a colour weakness, displaying a second graphic user interface to determine the degree of the colour weakness based on at least one gradation of brightness and saturation with respect to at least one colour, if it is determined that the user has the colour weakness, and setting a colour weakness mode corresponding to the determined degree of the colour weakness. Accordingly, the display apparatus determines whether a user has colour weakness and a degree of the colour weakness to adjust display characteristics of an image displayed on a display according to the degree of colour weakness.

## Description

The present invention relates to a display, particularly but not exclusively to a display and method for controlling the display so as to determine whether a user has defective colour vision and enable the characteristics of an image displayed on the display to be adjusted accordingly.

Colour vision refers to a person's ability to distinguish and recognise colours. If a person's colour vision is weak, they are referred to as having a colour vision weakness, or simply a colour weakness. More severe colour vision weakness is commonly referred to as colour blindness. Such a colour vision disability results in sufferers lacking the ability to recognise red, green, and/or blue (RGB) colours.

Colour vision disabilities may be inherited or be caused by disease. Eight percent of the population are estimated to suffer from an inherited colour vision disability.

Display devices, such as monitors or TVs, etc., display colour images and are increasingly becoming capable of reproducing colours close to true colour, as a result of developments in colour realisation techniques.

However, even if displays are capable of producing colour images close to true colour, users with colour vision disabilities may not correctly distinguish the images displayed on the display.

Some display manufacturers offer displays having functionality for individually adjusting red, green, and blue colours to help a user to correctly distinguish displayed images.

However, the colour adjustment functionality may cause inconvenience both for users with normal colour vision and for users with colour vision disabilities, since the displays can require adjustment to a normal mode for users with normal colour vision, and readjustment for users with colour vision disabilities.

Also, if a user does not know that he/she has a colour vision weakness, the user may use the display without correctly adjusting the colours.

It is desirable that a user is able to check whether he/she has a colour vision weakness without difficulty and adjust colour display conditions of an image displayed by a display apparatus according to the severity of the colour vision weakness.

Accordingly, the present invention provides a display apparatus and a control method thereof which determines whether a user has colour weakness and a degree of the colour weakness to adjust display characteristics of an image displayed on a display part corresponding to the degree of the colour weakness.

Additional aspects of the present invention will be set forth in part in the description which follows and, in part, will be obvious from the description, or may be learned by practice of the present invention.

The foregoing and/or other aspects of the present invention can be achieved by providing a method of controlling a display apparatus having a display part to display an image thereon, the method comprising displaying a first graphic user interface on the display part to determine whether a user has colour weakness, determining whether user has the colour weakness based on a first test signal which is input through the first graphic user interface, displaying a second graphic user interface having an information graphic user interface to display information regarding a process of determining a degree of the colour weakness, and at least one determination graphic user interface to determine the degree of the colour weakness based on at least one gradation of brightness and saturation with respect to at least one colour, on the display part if it is determined that the user has the colour weakness, determining the degree of the colour weakness based on a second test signal which is input through the second graphic user interface, and setting a colour weakness mode corresponding to the determined degree of the colour weakness.

The method may further comprise displaying a colour weakness set graphic user interface which is provided with a reset menu, a colour weakness mode menu to select at least one colour weakness mode and a colour weakness test menu, on the display part, wherein the displaying of the first graphic user interface on the display part is performed by selecting the colour weakness test menu of the displayed colour weakness set graphic user interface.

The method may further comprise displaying an image on the display part in a normal mode corresponding to selection of the reset menu of the displayed colour weakness set graphic user interface, and when the at least one colour weakness mode of the colour weakness mode menu is selected, displaying the image on the display part in a colour weakness mode which is set corresponding to the selected colour weakness mode.

The colour weakness set graphic user interface may further comprise a red control drag bar, a green control drag bar, and a blue control drag bar, and the method may further comprise selecting one of the at least one colour weakness mode of the colour weakness mode menu, activating the red control drag bar, the green control drag bar, and the blue control drag bar in a controllable state corresponding to the selection of the colour weakness mode, and displaying the image on the display part corresponding to control of at least one of the red control drag bar, the green control drag bar, and the blue control drag bar, as the selected colour weakness mode.

The colour weakness set graphic user interface may further comprise an initialization menu, and further comprises initializing the colour weakness mode corresponding to the selection of the initialization menu.

The first graphic user interface may comprise a colour weakness test image and a test input window to input the first test signal corresponding to the colour weakness test image.

The determining of whether the user has the colour weakness may comprise displaying at least two colour weakness test images on the first graphic user interface sequentially, inputting the first test signal in the test input window corresponding to each of the sequentially displayed colour weakness test images, and determining that the user has the colour weakness if the colour weakness test images and the first test signal are not identical to each other more than a predetermined number of times.

The first graphic user interface may further comprise a next menu, wherein the displaying of the at least two colour weakness test images sequentially comprises displaying a next one of the at least two colour weakness test images on the first graphic user interface whenever the next menu is selected.

The method may further comprise displaying a normal vision information display window on the display part to display information representing that the user has a normal colour vision if the colour weakness test images and the first test signal are identical to each other more than the predetermined number of times.

The displaying of the second graphic user interface on the display part may be performed when it is determined that the user has the colour weakness.

The setting of the colour weakness mode corresponding to the determined degree of the colour weakness may comprise displaying a mode set window which includes at least one determination mode menu corresponding to the at least one colour weakness mode of the colour weakness mode menu of the colour weakness set graphic user interface, on the display part when the degree of the colour weakness is determined through the determination graphic user interface, and setting the colour weakness mode corresponding to the determined degree of the colour weakness as the at least one colour weakness mode of the colour weakness mode menu corresponding to the at least one determination mode menu if the at least one determination mode menu is selected.

The foregoing and/or other aspects of the present invention are also achieved by providing a method of controlling a display apparatus comprising receiving first input signals and determining whether a user has a colour weakness based on the first input signals, receiving second input signals and determining a degree of the colour weakness based on the second input signals if it is determined that the user has the colour weakness, and adjusting display characteristics of the display apparatus according to the determined degree of the colour weakness.

The foregoing and/or other aspects of the present invention are also achieved by providing a display apparatus comprising a display part to display an image thereon, an image processor to convert an image signal into a signal which is displayable by the display part, and a controller to control the display part to display a first graphic user interface to determine whether a user has colour weakness and a second graphic user interface having an information graphic user interface to display information regarding a process of determining a degree of the colour weakness and at least one determination graphic user interface to determine the degree of the colour weakness based on at least one gradation of brightness and saturation with respect to at least one colour, to determine whether the user has the colour weakness based on a first test signal which is input through the first graphic user interface, to determine the degree of the colour weakness based on a second test signal which is input through the second graphic user interface, and to set a colour weakness mode corresponding to the determined degree of colour weakness.

The controller may comprise a first controller which is provided in an external device to apply the image signal to the image processor, to apply graphic user interface image signals corresponding to the first graphic user interface and the second graphic user interface to the image processor, and to determine whether the user has the colour weakness and the degree of the colour weakness according to the first test signal and the second test signal, and a second controller to control the image processor to change colour display characteristics of the image displayed on the display part according to a control signal from the first controller corresponding to the determined degree of the colour weakness.

The first and second controllers may communicate with each other through a display data communication (DDC) communication line.

The controller may control the display part to display a colour weakness set graphic user interface which includes a reset menu, a colour weakness test menu, and a colour weakness mode menu to select at least one colour weakness mode, and to display the first graphic user interface when the colour weakness test menu is selected.

The controller may control the image processor to set colour display characteristics of the image displayed on the display part to a predetermined normal mode when the reset menu is selected, and may control the image processor to set the colour display characteristics of the image displayed on the display part to correspond to the selected at least one colour weakness mode when the at least one colour weakness mode of the colour weakness mode menu is selected.

The colour weakness set graphic user interface may further comprise a red control drag bar, a green control drag bar, and a blue control drag bar, and the controller may activate the red control drag bar, the green control drag bar, and the blue control drag bar to be in a controllable state when one of the at least one colour weakness mode of the colour weakness mode menu is selected, and may control the image processor to adjust display characteristics of the image displayed on the display part corresponding to control of at least one of the red control drag bar, the green control drag bar, and the blue control drag bar.

The colour weakness set graphic user interface may further comprise an initialization menu, and the controller may initialize a preset colour weakness mode according to selection of the initialization menu if the initialization menu is selected.

The first graphic user interface may include at least two colour weakness test images sequentially displayed on the display part, and the controller may determine that the user has the colour weakness if the first test signal input corresponding to the sequentially displayed colour weakness test images is not identical with the colour weakness test images a predetermined number of times.

The controller may control the display part to display an information display window to display information that a user has a normal colour vision if the first test signal is identical with the colour weakness test images more than the predetermined number of times.

The controller may control the display part to display a mode set window which is provided with at least one determination mode menu corresponding to the at least one colour weakness mode of the colour weakness mode menu of the colour weakness set graphic user interface when the degree of the colour weakness is determined through the determination graphic user interface, and may set the colour weakness mode corresponding to the determined degree of the colour weakness as the at least one colour weakness mode of the colour weakness mode menu corresponding to the at least one determination mode menu if the at least one determination mode menu is selected.

The foregoing and/or other aspects of the present invention are also achieved by providing a display apparatus comprising a display part to display first and second graphic user interfaces, and a controller to determine whether a user has a colour weakness based on the displayed first graphic user interface, to determine a degree of the colour weakness based on the displayed second graphic user interface when it is determined that the user has the colour weakness, and to adjust display characteristics of the display part according to the determined degree of the colour weakness.

The foregoing and/or other aspects of the present invention are also achieved by providing a display apparatus comprising a display part to display a plurality of test images thereon to test whether a user has a colour weakness and to display a plurality of gradations of each of a plurality of colours thereon to test a degree of the colour weakness, a user input part to generate first signals corresponding to the plurality of test images displayed on the display, to arrange the plurality of gradations of each of the plurality of colours according colour saturation, and to generate second signals corresponding to the arrangement of the gradations of each of the plurality of colours, and a controller to compare the first signals to the test images to determine whether the user has the colour weakness and to compare the second signals with a predetermined normal arrangement of the gradations of each of the plurality of colours to determine the degree of the colour weakness for each of the plurality of colours when is determined that the user has the colour weakness.

The foregoing and/or other aspects of the present invention are also achieved by providing a display system, comprising a display unit comprising a display part to display an image thereon and a display controller to control display characteristics of the display part, and a computer communicating with the display unit and comprising a control unit to control the display unit to display a first graphic user interface and a second graphic user interface, to determine whether a user has a colour weakness based on the displayed first graphic user interface, to determine a degree of the colour weakness based on the displayed second graphic user interface when it is determined that the user has the colour weakness, and to control the display controller to adjust the display characteristics of the display part according to the determined degree of the colour weakness.

The foregoing and/or other aspects of the present invention are also achieved by providing a computer readable recording medium having executable codes to perform a method of controlling a display apparatus having a display part to display an image thereon, the method comprising displaying a first graphic user interface on the display part to determine whether a user has colour weakness, determining whether the user has the colour weakness based on a first test signal which is input through the first graphic user interface, displaying a second graphic user interface having an information graphic user interface to display information regarding a process of determining a degree of the colour weakness, and at least one determination graphic user interface to determine the degree of the colour weakness based on at least one gradation of brightness and saturation with respect to at least one colour, on the display part if it is determined that the user has the colour weakness, determining the degree of the colour weakness based on a second test signal which is input through the second graphic user interface, and setting a colour weakness mode corresponding to the determined degree of the colour weakness.

The foregoing and/or other aspects of the present invention are also achieved by providing a computer readable recording medium having executable codes to perform a method of controlling a display apparatus, the method comprising receiving first input signals and determining whether a user has a colour weakness based on the first input signals, receiving second input signals and determining a degree of the colour weakness based on the second input signals if it is determined that the user has the colour weakness, and adjusting display characteristics of the display apparatus according to the determined degree of the colour weakness.

Embodiments of the invention will now be described, by way of example, with reference to the accompanying drawings, in which:
Figure 1 is a block diagram illustrating a display apparatus according to an embodiment of the present invention;
Figures 2 to 9 illustrate examples of graphic user interfaces displayed by the display apparatus of Figure 1; and
Figure 10 is a block diagram illustrating a display apparatus according to another embodiment of the present invention.

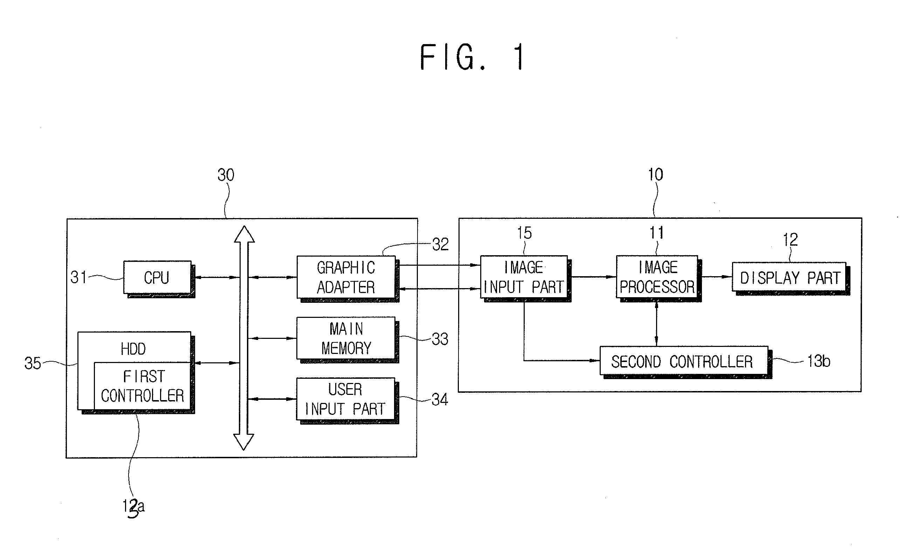

Figure 1 illustrates a display apparatus 10 and computer 30 according to an embodiment of the present invention. Referring to Figure 1, the display apparatus 10 and computer 30 include a control arrangement including a first controller 13a and a second controller 13b. The display apparatus 10 comprises a display part, also referred to as a screen or display, 12, an image input part 15, an image processor 11 and the second controller 13b.

The display part 12 receives a signal from the image processor 11 causing it to display an image. The display part 12 may be provided as a flat panel display, such as a liquid crystal display (LCD) panel or a plasma display panel (PDP), or a cathode ray tube (CRT), but the present invention is not limited thereto.

The image processor 11 converts an image signal input through the image input part 15 into a signal which is displayable by the display part 12, and outputs the signal to the display part 12. The image processor 11 may comprise a scaler to scale the image signal and a signal converter to convert the input image signal into a format which is displayable, e.g., a digital RGB signal. The signal converter may comprise a transition minimized differential signaling (TMDS) receiver, an A/D converter, a video decoder, a tuner, etc., corresponding to a video format of the image signal input through the image input part 15.

The image input part 15 may comprise a digital connection terminal, such as a digital visual interface (DVI) connector, a D-Sub connector, an antenna, an S-video terminal, a component terminal, etc., corresponding to the video format of the image signal input through the image input part 15.

As illustrated in the embodiment of Figure 1, an external device, e.g. the computer 30, can be connected to the DVI connector or the D-Sub connector of the image input part 15 of the display apparatus 10 to input the image signal to the display apparatus 10.

The computer 30 comprises a CPU 31, a graphics adapter 32, a main memory 33, a hard disk drive (HDD) 35, and a user input part 34. The first controller 13a can be stored on the HDD 35. The first controller 13a can be a control program loaded to the main memory 33 from the HDD 35 and executable to perform a predetermined function upon a request by a user.

The user input part 34 may be provided in various forms, such as a keyboard, a mouse, etc., to apply a key signal to the computer 30 corresponding to the user's control.

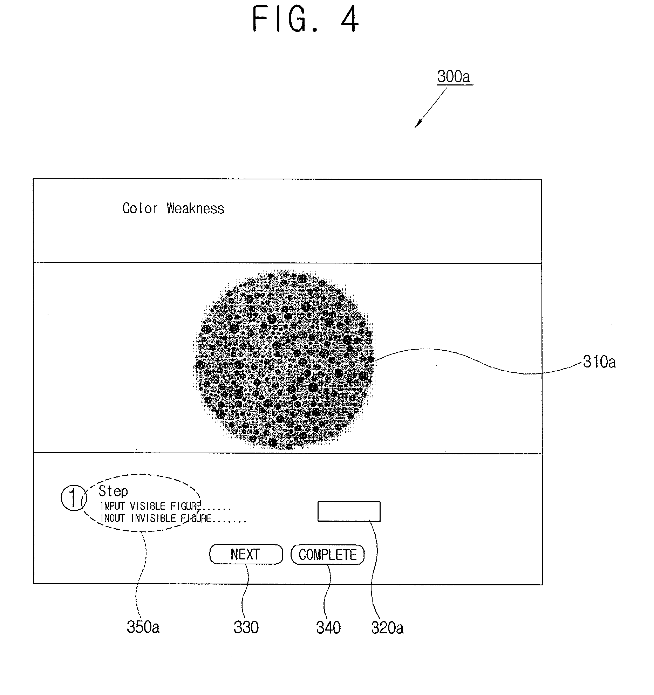

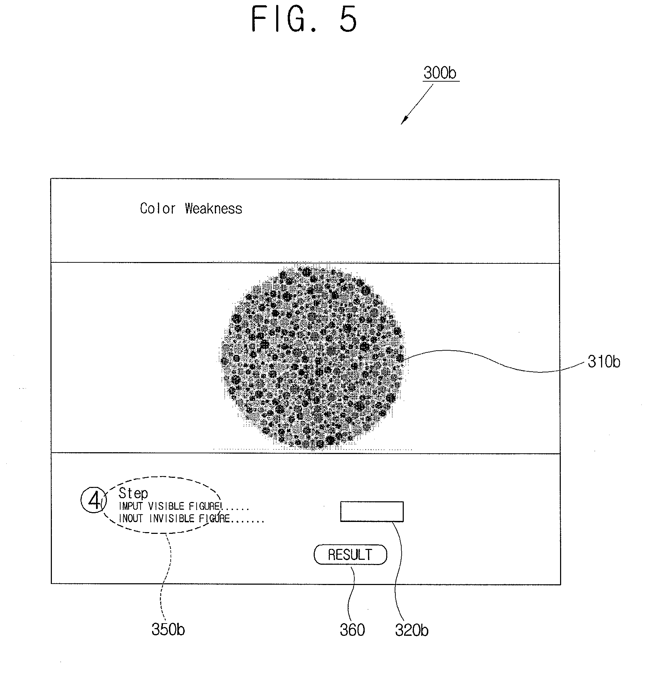

The first controller 13a controls the display apparatus 10 to display one or more first graphic user interfaces 300a and 300b (see Figures 4 and 5), and a second graphic user interface on the display part 12. The first controller 13a determines whether the user has colour weakness based on one or more first test signals input through the one or more first graphic user interfaces 300a and 300b (see Figures 4 and 5), and a degree of the colour weakness based on a second test signal input through the second graphic user interface. The second controller 13b sets a colour weakness mode of the display apparatus 10 corresponding to the degree of the colour weakness determined by the first controller 13a.

As described above, the controller arrangement of the display apparatus 10 and computer 30 illustrated in the embodiment of Figure 1 comprises the first controller 13a which is provided in the computer 30, and the second controller 13b which is provided in the display apparatus 10.

The first controller 13a is operated by the computer 30 and transmits image signals corresponding to the first graphic user interfaces 300a and 300b (see Figures 4 and 5) and the second graphics user interface, to the display apparatus 10. The first controller 13a determines whether the user has a colour weakness and the degree of the colour weakness according to first and second test signals received from the user via the graphic user interfaces.

The second controller 13b controls the image processor 11 to change the colour display characteristics of the image displayed on the screen 12 according to the degree of the colour weakness determined by the first controller.

The first controller 13a and the second controller 13b can communicate with each other through the graphics adaptor 32 using a DDC communication line, but the present invention is not limited thereto. For example, the first controller 13a and the second controller 13b can alternatively communicate with each other wirelessly.

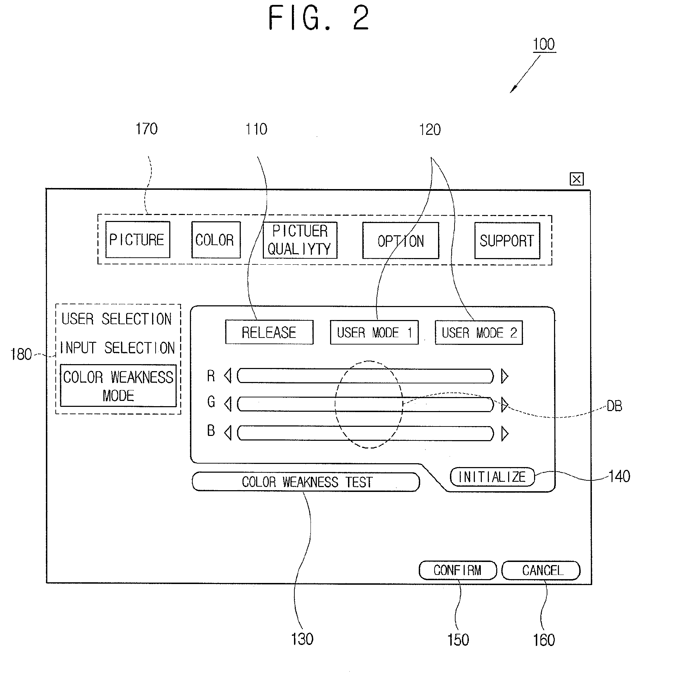

Figures 2 to 9 illustrate graphic user interfaces displayable on the screen 12 under the control of the first controller 13a. Referring to Figures 2 through 9, a process of controlling the display apparatus 10 according to an embodiment of the present invention will be described in detail.

When the first controller 13a is operated by the computer 30, the first controller 13a controls the display apparatus 10 to display a colour weakness graphic user interface 100 on the display part 12, as illustrated in Figure 2.

The user interfaces of the present invention comprise a number of selectable options, in this case in the form of on-screen buttons selectable by a user using a mouse. In particular, the colour weakness graphic user interface 100 includes a reset button 110, a colour weakness test button 130, and at least one colour weakness mode button 120 to select at least one colour weakness mode. For example, as illustrated in Figure 2, the colour weakness graphic user interface 100 is provided with two colour weakness mode buttons 120 corresponding to two colour weakness modes, user mode 1 and user mode 2, but the number thereof is not limited thereto and can be, for example, three or more.

If the user selects the reset button 110 using the user input part 34, the first controller 13a applies a reset control signal corresponding to selection of the reset button 110 to the second controller 13b through the DDC communication line. In response to the reset control signal, the second controller 13b controls the image processor 11 to set the colour display characteristics of images displayed on the display part 12 to a predetermined normal mode.

If the user selects one of the colour weakness mode buttons 120 using the user input 34, the first controller 13a applies a control signal corresponding to a preset colour weakness mode corresponding to the selected colour weakness mode button 120 to the second controller 13b through the DDC communication line. In response to the control signal, the second controller 13b controls the image processor 11 to set the colour display characteristics of images displayed on the display part 12 to the corresponding colour weakness mode.

The colour weakness graphic user interface 100 may further comprise a main function menu 170 and a sub function menu 180. The main function menu 170 includes selectable menus that group together similar functions to adjust the display characteristics of the the display part 12. For example, as illustrated in Figure 2, the main function menu 170 can include a picture menu, a colour menu, a picture quality menu, an option menu, and a support menu. The sub function menu 180 is provided to enable a user to select a sub function with respect to each of the menus provided in the main function menu 170.

The colour weakness graphic user interface 100 can be displayed on the display part 12, as illustrated in Figure 2, when the colour weakness mode is selected from the sub function menu 180 of the option menu of the main function menu 170.

The colour weakness graphic user interface 100 may further comprise an initialization button 140. If the user selects the initialization button 140 using the user input part 34, the first controller 13a initializes the colour weakness mode which is set corresponding to the respective colour weakness mode buttons 120. The initialization of the colour weakness mode may revert the display 12 to a factory mode or the level of respective colours to one or more preset values.

The colour weakness graphic user interface 100 may further comprise a drag bar DB including a red control drag bar, a green control drag bar, and a blue control drag bar. If the user selects one of the colour weakness mode buttons 120, the first controller 13a activates the red control drag bar, the green control drag bar, and the blue control drag bar corresponding to the selection of the colour weakness mode button 120 to be adjustable through the user input part 34.

The user can then control at least one of the red control drag bar, the green control drag bar, and the blue control drag bar using the user input part 34 to adjust a level of the corresponding colour according to the control of the corresponding drag bar. For instance, the intensity level of the colour can be adjusted in accordance with the position of a marker on the drag bar. When the user controls one of the red, green, and blue control drag bars, the first controller 13a applies a control signal according to the control of the drag bar to the second controller 13b through the DDC communication line, and the second controller 13b adjusts the level of the corresponding colour adjusted according to the control signal. That is, the second controller 13b controls the image processor 11 to adjust the level of the corresponding colour according to the control signal of the first controller 13a.

The first controller 13a is, in the present example, arranged to store the levels of the colours adjusted using the control of the drag bar as the colour weakness mode of the selected colour weakness mode button 120. If the user selects the corresponding colour weakness mode button 120 in the future, the first controller 13a then outputs a control signal corresponding to the colour weakness mode of the selected colour weakness mode button 120 to the second controller 13b. The second controller 13b controls the image processor 11 to display the image on the screen 12 using the levels of the colours stored as the selected colour weakness mode.

The colour weakness mode button 120 can also include a confirm button 150 and a cancel button 160. The confirm button 150 can be selected to complete the colour level adjusting process after storing the colour levels set using the red, green, and blue control drag bars of the colour weakness graphic user interface 100. The cancel button 160 can be selected to complete the level adjusting process without storing the conditions set through the red, green, and blue control drag bars of the colour weakness graphic user interface 100.

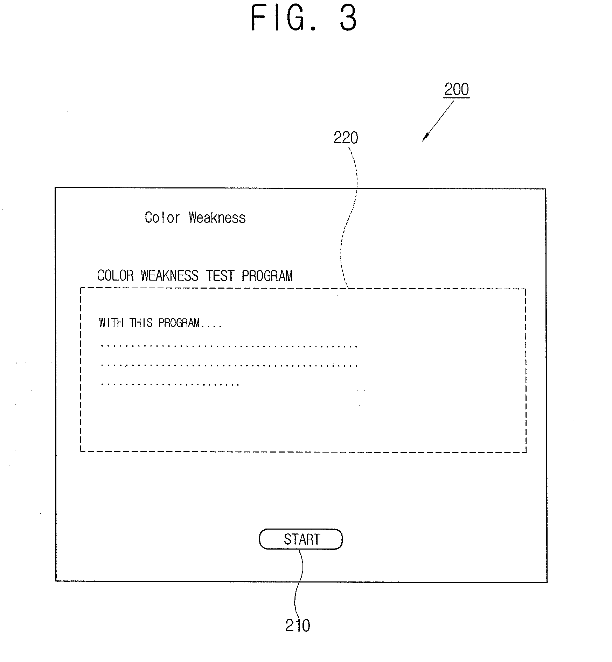

If the colour weakness test button 130 is selected, the first controller 13a controls the display apparatus 10 to display an initial graphic user interface 200, as illustrated in Figure 3, or the first graphic user interfaces 300a and 300b, as illustrated in FIGS 4 and 5, on the display part 12.

Referring to Figure 3, the first controller 13a may control the display apparatus 10 to display the initial graphic user interface 200, which displays information 220 regarding the process of colour weakness determination on the display part 12. The initial graphic user interface 200 is provided with a start button 210 to start the colour weakness determination process.

When the start button 210 of the initial graphic user interface 200 is selected, the first controller 13a controls the display apparatus 10 to sequentially display the one or more first graphic user interfaces 300a and 300b, as illustrated in Figures 4 and 5. It is also possible that the first controller 13a is configured to control the display apparatus 10 to sequentially display the one or more first graphic user interfaces 300a, 300b in response to the selection of the colour weakness test button 130 of the colour weakness graphic user interface 100, instead of displaying the initial graphic user interface 200 before the first user interfaces 300a and 300b.

Referring to Figures 4 and 5, the one or more first user interfaces 300a and 300b are sequentially displayed at the display part 12. The respective first graphic user interfaces 300a and 300b may comprise colour weakness test images 310a and 310b, and test input windows 320a and 320b. The first graphic user interfaces 300a and 300b display the colour weakness test images 310a and 310b to determine whether the user has a colour weakness. The user can input figures or characters in the test input windows 320a and 320b corresponding to figures and characters that they see displayed on the colour weakness test images 310a and 310b. The figures or characters input to the test windows 320a and 320b are received by the first controller 13a as first test signals and, since these will be dependent on the accuracy of a user's colour vision, they can used by the first controller 13a to determine whether the user has a colour vision weakness.

The first controller 13a determines whether the user has a colour vision weakness based on whether the respective sequentially displayed test images 310a and 310b match the corresponding first test signals. For example, if the colour weakness test images 310a and 310b do not match the first test signal a predetermined number of times in a test conducted by sequentially displaying first graphic user interfaces 300a, 300b, the first controller 13a determines that the user has a colour vision weakness. If the colour weakness test images 310a and 310b are identical with the first test signals the predetermined number of times in the test, the first controller 13a determines that the user has normal colour vision.

As illustrated in Figures 4 and 5, there are provided two tests (i.e., two first graphic user interfaces 300a and 300b each including the respective colour weakness test image 310a and 310b) for determining if the colour weakness test images 310a and 310b are identical with the first test signals. However, the present invention is not limited thereto, and there may alternatively be provided three or more tests, for instance depending on the accuracy of the tests. Furthermore, the first controller 13a may determine that the user has a colour weakness when the colour weakness test images 310a and 310b are not identical with the first test signals, one, two, or more times.

As illustrated in Figure 4, the first graphic user interface 300a may include a next button 330. When the next button 330 is selected, the first controller 13a controls the display apparatus 10 to display the next sequentially displayed first graphic user interface 300b to move to the next test in the colour weakness determination process.

As illustrated in Figure 4, the first graphic user interface 300a can include a complete button 340 to select completion of the colour weakness determination process. If the complete button 340 is selected, the first controller 13a completes the process of determining whether the user has the colour weakness. The first graphic user interfaces 300a and 300b can also include information display blocks 350a and 350b to provide information to describe the currently displayed test of the colour weakness determination process.

As illustrated in Figure 5, a final one of the sequentially displayed first graphic user interfaces 300b can include a result button 360 to display results of the colour weakness determination process conducted through the first graphic user interfaces 300a and 300b. When the result button 360 is selected, the first controller 13a provides the results of the colour weakness determination to the user by controlling the display apparatus 10 to display the results on the screen 12.

For example, if the colour weakness test images 310a and 310b are identical with the first test signals more than the predetermined number of times, the first controller 13a controls the display apparatus 10 to display information notifying the user that the user has normal colour vision, e.g., a normal vision information display window (not shown) comprising a sentence such as "you have no colour weakness", on the screen 12. It is possible that the normal vision information display window comprises a confirm button. When the user selects the confirm button, the first controller 13a can complete the process of determining the colour weakness.

If the colour weakness test images 310a and 310b are not identical with the first test signals more than the predetermined number of times, i.e., if the user is determined to have the colour weakness, the first controller 13a controls the display apparatus 10 to display a second graphic user interface on the screen 12 to determine a degree of the colour weakness.

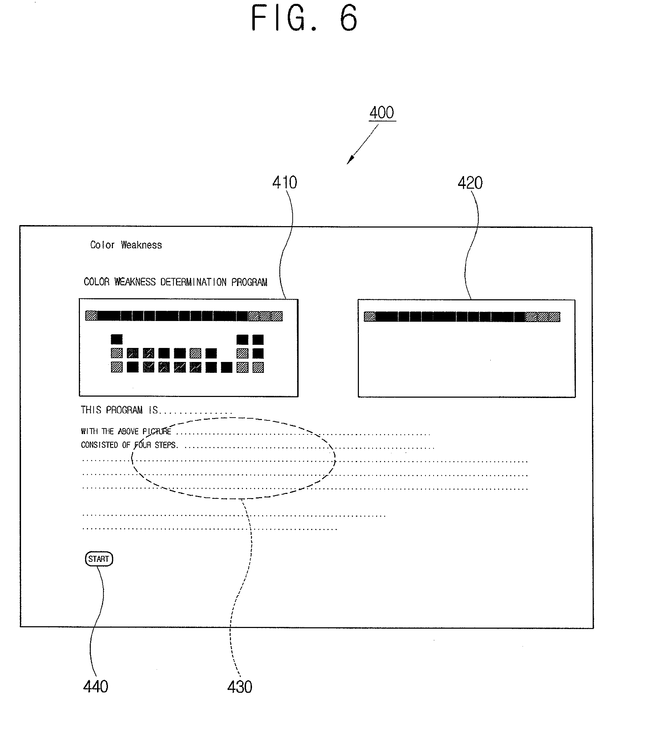

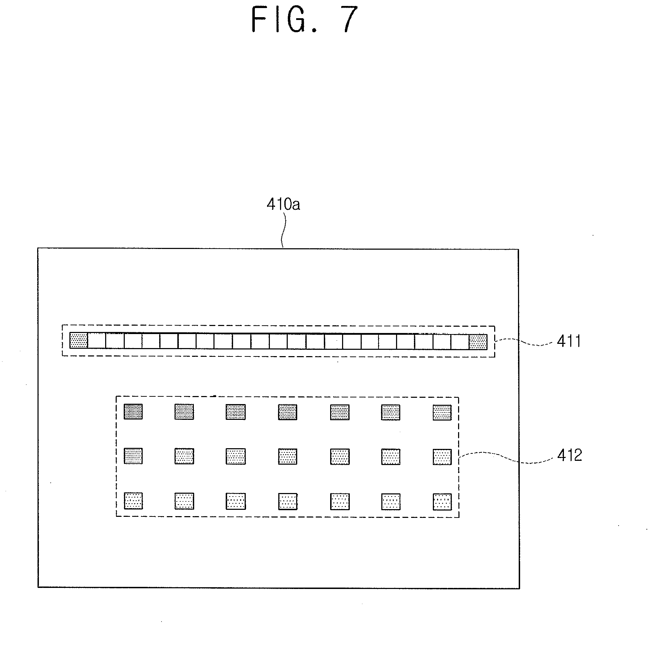

The second graphic user interface may comprise an information graphic user interface 400 (see Figure 6) to display information regarding a process of determining the degree of the colour weakness, and a determination graphic user interface 410a (see Figure 7) to determine the degree of the colour weakness.

Figure 6 illustrates an example of the information graphic user interface 400. Referring to Figure 6, the information graphic user interface 400 may comprise a first gradation block 410 and a second gradation block 420.

The first gradation block 410 displays a gradation range block 411 (see Figure 7) to be initially displayed on the determination graphic user interface 410a, and a plurality of colour gradation boxes 412 to be arranged by the user in the gradation range block 411. The second gradation block 420 displays the colour gradation boxes 412 of the first gradation block 410 arranged correctly in the gradation range block 411. That is, the second gradation block 420 displays the arrangement of the colour gradation boxes 412 in the gradation range block 411 corresponding to the user having the normal colour vision.

The information graphic user interface 400 is also provided with an information block 430 to display information regarding the process of determining the degree of the colour weakness, e.g., information about arranging the gradation boxes 412 in the first gradation block 410 in the arrangement displayed in the second gradation block 420.

When the user selects a start button 440 which is provided in the information graphic user interface 400, the first controller 13a controls the display apparatus 10 to sequentially display the determination graphic user interface 410a with respect to the plural colours (i.e., red, green, and blue). The user then arranges each respective determination graphic user interface 410a in the arrangement of the second gradation block 420 of the information graphic user interface 400. The first controller 13a receives the colour arrangement of each respective determination graphic user interface 410a as a second test signal and determines the degree of the colour weakness with respect to the corresponding colour.

Figure 7 illustrates an example of the determination graphic user interface 410a. Referring to Figure 7, the determination graphic user interface 410a has a layout corresponding to the first gradation block 410 of the information graphic user interface 400. The determination graphic user interface 410a is provided with the gradation range block 411 and the plurality of colour gradation boxes 412 to be arranged in the gradation range block 411. The user arranges the colour gradation boxes 412 in the gradation range block 411 according to a degree of colour saturation. The first controller 13a determines the degree of colour weakness with respect to the corresponding colour according to the arrangement by the user. That is, the first controller 13a receives the second control signal corresponding to the arrangement of the colour gradation boxes 412 of each colour and compares the second control signal with the correct arrangement of the colour gradation boxes 412 as displayed in the second gradation box 420 of the information graphic user interface 400. The first controller 13a determines the degree of the colour weakness with respect to each colour based on the comparison.

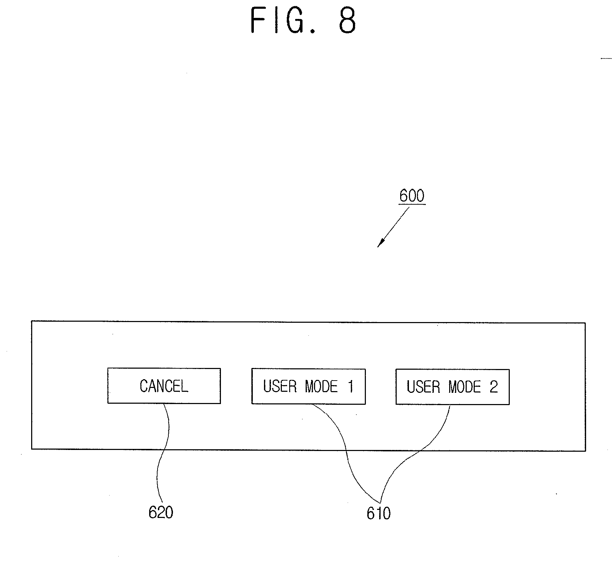

When the process of determining the degree of the colour weakness conducted through the determination graphic user interface 410a is completed, the first controller 13a controls the display apparatus 10 to display a mode set window 600 (see Figure 8) which is provided with one or more determination mode buttons 610 corresponding to the respective colour weakness mode buttons 120 of the colour weakness graphic user interface 100, on the screen 12.

If the user selects one of the determination mode buttons 610, the first controller 13a sets a colour weakness mode corresponding to the determined degree of the colour weakness as the colour weakness mode of the colour weakness mode button 120 corresponding to the selected determination mode button 610. That is, the first controller 13a determines colour display characteristics of the display part 12 corresponding to the determined degree of the colour weakness and stores the determined colour display characteristics as the colour weakness mode of the colour weakness mode button 120 corresponding to the selected determination mode button 610. Accordingly, when the user selects the corresponding colour weakness mode button 120 of the colour weakness graphic user interface 100, the first controller 13a applies the control signal corresponding to the colour weakness mode corresponding to the determined degree of the colour weakness to the second controller 13b. In response to the control signal, the second controller 13b controls the image processor 11 to display the image displayed on the display part 12 using the corresponding colour weakness mode. That is, the second controller 13b controls the image processor 11 to adjust the colour display characteristics of the display part 12 to the colour display characteristics stored as the colour weakness mode of the corresponding colour weakness mode button 120. The colour display characteristics can include, for instance, the saturation, tone and/or brightness of one or more displayed colours.

If the user selects a cancel button 620 which is provided in the mode set window 600, the process of determining the degree of the colour weakness is completed without setting or storing the colour weakness mode corresponding to the determined degree of the colour weakness.

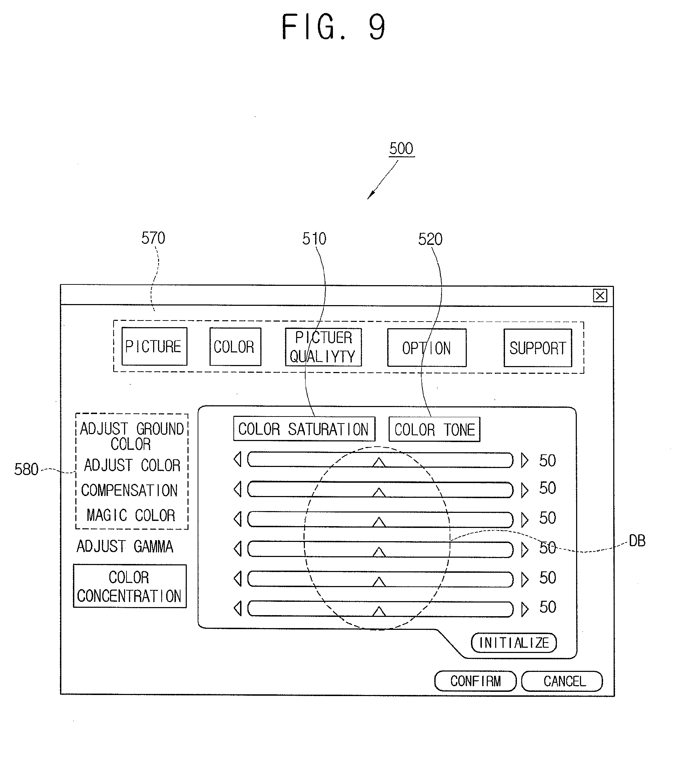

Figure 9 illustrates the colour weakness graphic user interface 500 as displayed on the display part 12 if the user selects the colour button of the main function menu 170 and a colour concentration button of the sub function menu 180.

Referring to Figure 9, the colour weakness graphic user interface 500 includes a colour saturation button 510 for viewing colour saturation options and a colour tone button 520 for viewing colour tone options. The user may use colour saturation drag bars DB of the colour saturation options and colour tone drag bars DB of the colour tone options to adjust the colour saturation and colour tone of the image displayed on the screen 12. If the second controller 13b controls the image processor 11 to display the image on the screen 12 in a colour weakness mode corresponding to a selected one of the colour weakness mode buttons 120, the first controller 13a may control the drag bars DB of the colour saturation options 510 and the colour tone options 520 to be displayed in a non-controllable state. Accordingly, if the user tries to adjust the drag bars DB, the first controller 13a can display an information window (not shown) which overlaps with the colour weakness graphic user interface 500 and provides information informing the user that the colour weakness mode is already set.

As illustrated in Figure 1, the computer 30 is connected with a single display apparatus 10. However, if the computer 30 is connected with two or more display apparatuses 10 and the display apparatuses 10 support the functions described above, the first controller 13a may set the colour weakness mode individually with respect to each display apparatus 10. If the colour weakness mode is set in a single display apparatus 10, the first controller 13a may also apply the set colour weakness mode to all of the display apparatuses 10. For example, if a user sets the colour weakness mode corresponding to one of the colour weakness mode buttons 120 of the colour weakness graphic user interface 100 and selects the corresponding colour weakness mode button 120, the colour weakness mode may be applicable to all of the display apparatuses 10. The first controller 13a then applies the control signal corresponding to the selected colour weakness mode to the respective display apparatuses 10. The second controller 13b provided in each display apparatus 10 controls the respective image processor 11 to display the image displayed on the display part 12 of the respective display apparatus 10 in the corresponding colour weakness mode.

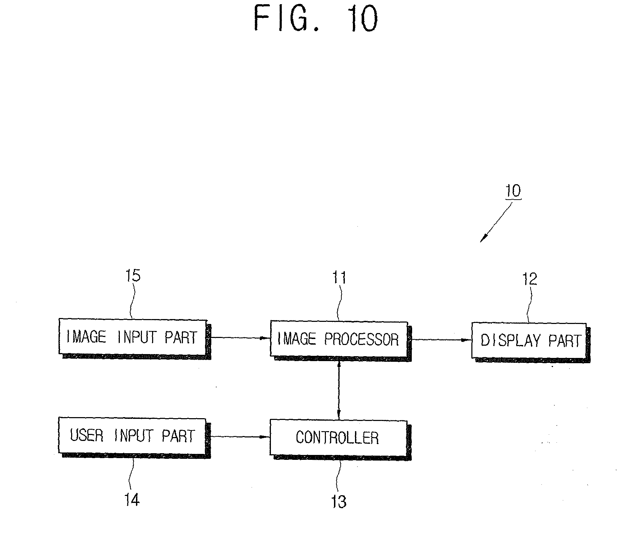

Figure 10 is a block diagram illustrating a display apparatus 10' according to another embodiment of the present invention. Referring to Figure 10, the display apparatus 10' comprises an image input part 15, an image processor 11, a display part 12, a user input 14, and a controller 13.

The display apparatus 10' of the embodiment of Figure 10 differs from the display apparatus 10 of the embodiment of Figure 1 in that the controller 13 provided in the display apparatus 10' of Figure 10 can perform all functions of the first and second controllers 13a and 13b of the computer 30 and the display apparatus 10 of Figure 1. Accordingly, the controller 13 provided in the display apparatus 10' controls the display apparatus to display the first graphic user interfaces 300a and 300b and the second graphic user interface on the display part 12, and determines whether a user has a colour vision weakness and the degree of the colour vision weakness. The controller 13 also controls the image processor 11 to adjust the display conditions of the image displayed on the display part 12 according to the determined degree of the colour weakness.

The graphics user interfaces described above, such as the first graphic user interfaces 300a and 300b and the second graphic user interface, may be displayed on the display part 12 by an on screen display (OSD) generator (not shown), which is provided in the image processor 11.

The user input part 14 of the display apparatus 10' of the embodiment of Figure 10 may be provided as a control button which is disposed on a surface of the display apparatus 10' or a remote controller.

It is possible for the present invention to be realized on a computer-readable recording medium as a computer-readable code. Computer-readable recording mediums include many types of recording devices that store computer system-readable data. ROMs, RAMs, CD-ROMs, magnetic tapes, floppy discs, optical data storage, etc. are used as computer-readable recording mediums. Computer-readable recording mediums can also be realized in the form of carrier waves (e.g., transmission via Internet).

Although a few embodiments of the present invention have been shown and described, it will be appreciated by those skilled in the art that changes may be made in these embodiments without departing from the principles of the invention, the scope of which is defined in the appended claims.

## Claims

1. A method of controlling a display comprising:
providing a user of the display with an interactive test to determine whether the user has defective colour vision; and, in response to the determination,
automatically adjusting output characteristics of the display.

2. A method according to claim 1, wherein the step of providing a user of the display with an interactive test comprises:
receiving first input signals and determining whether a user has a colour weakness based on the first input signals; and
receiving second input signals and determining a degree of the colour weakness based on the second input signals if it is determined that the user has the colour weakness; and wherein the step of automatically adjusting output characteristics of the display comprises:
automatically adjusting display characteristics of the display according to the determined degree of the colour weakness.

3. A method according to claim 2, wherein the receiving of the first input signals and determining whether the user has a colour weakness based on the first input signals comprises:
sequentially displaying a plurality of test images;
receiving the first input signals, each corresponding to one of the test images; and
comparing the received first input signals with the displayed test images to determine whether the user has the colour weakness.

4. A method according to claim 3, wherein the comparing of the received first input signals with the displayed test images comprises:
determining that the user has the colour weakness when a predetermined number of the received first input signals are not identical to the corresponding test images.

5. A method according to claim 2, 3 or 4, wherein the receiving of the second input signals and determining of the degree of the colour weakness based on the second input signals comprises:
displaying a plurality of gradations of each of a plurality of colours to be arranged by the user in order of colour saturation;
receiving the second test signals, each corresponding to the arrangement of the gradations of one of the colours by the user; and
comparing the second test signals with a predetermined normal arrangement of the gradations of each colour to determine the degree of the colour weakness for each colour.

6. The method according to any one of claims 2 to 5, wherein the adjusting of the display characteristics of the display according to the determined degree of the colour weakness comprises:
determining level values of each of a plurality of colours of the display to compensate for the determined degree of the colour weakness; and
storing the determined level values as a colour weakness mode selectable to set levels of each of the plurality of colours to the determined level values.

7. A method according to any one of claims 2 to 6, further comprising:
setting the display characteristics of the display to a predetermined normal mode when it is determined that the user does not have the colour weakness.

8. A method according to any preceding claim, further comprising:
displaying a first graphic user interface on the display to determine whether a user has a colour weakness;
determining whether the user has the colour weakness based on a first test signal which is input through the first graphic user interface;
displaying a second graphic user interface having an information graphic user interface to display information regarding a process of determining a degree of the colour weakness, and at least one determination graphic user interface to determine the degree of the colour weakness based on at least one gradation of brightness and saturation with respect to at least one colour, on the display if it is determined that the user has the colour weakness;
determining the degree of the colour weakness based on a second test signal which is input through the second graphic user interface; and
setting a colour weakness mode for the display corresponding to the determined degree of the colour weakness.

9. A method according to claim 8, further comprising:
displaying a colour weakness set graphic user interface which is provided with a reset menu, a colour weakness mode menu to select at least one colour weakness mode and a colour weakness test menu, on the display, wherein
the displaying of the first graphic user interface on the display is performed by selecting the colour weakness test menu of the displayed colour weakness set graphic user interface.

10. A method according to claim 9, further comprising:
displaying an image on the display in a normal mode corresponding to selection of the reset menu of the displayed colour weakness set graphic user interface; and
when the at least one colour weakness mode of the colour weakness mode menu is selected, displaying the image on the display in a colour weakness mode which is set corresponding to the selected colour weakness mode.

11. A method according to claim 9 or 10, wherein the colour weakness set graphic user interface further comprises a red control drag bar, a green control drag bar, and a blue control drag bar, the method further comprising:
selecting one of the at least one colour weakness mode of the colour weakness mode menu;
activating the red control drag bar, the green control drag bar, and the blue control drag bar in a controllable state corresponding to the selection of the colour weakness mode; and
displaying the image on the display corresponding to the control of at least one of the red control drag bar, the green control drag bar, and the blue control drag bar, as the selected colour weakness mode.

12. The method according to any one of claims 9 to 11, wherein the colour weakness set graphic user interface further comprises an initialization menu, the method further comprising:
initializing the colour weakness mode corresponding to the selection of the initialization menu.

13. A method according to any one of claims 8 to 12, wherein the first graphic user interface comprises a colour weakness test image and a test input window to input the first test signal corresponding to the colour weakness test image.

14. A method according to claim 13, wherein the determining of whether the user has the colour weakness comprises:
displaying at least two colour weakness test images on the first graphic user interface sequentially;
inputting the first test signal in the test input window corresponding to each of the sequentially displayed colour weakness test images; and
determining that the user has the colour weakness if the colour weakness test images and the first test signal are not identical to each other more than a predetermined number of times.

15. A method according to claim 14, wherein the first graphic user interface further comprises a next menu, wherein
the displaying of the at least two colour weakness test images sequentially comprises displaying a next one of the at least two colour weakness test images on the first graphic user interface whenever the next menu is selected.

16. A method according to any one of claims 8 to 15, further comprising:
displaying a normal vision information display window on the display to display information representing that the user has a normal colour vision if the colour weakness test images and the first test signal are identical to each other more than the predetermined number of times.

17. A method according to any one of claims 8 to 16, wherein the displaying of the second graphic user interface on the display is performed when it is determined that the user has the colour weakness.

18. A method according to any one of claims 8 to 17, wherein the setting of the colour weakness mode corresponding to the determined degree of the colour weakness comprises:
displaying a mode set window which includes at least one determination mode menu corresponding to the at least one colour weakness mode of the colour weakness mode menu of the colour weakness set graphic user interface, on the display when the degree of the colour weakness is determined through the determination graphic user interface; and
setting the colour weakness mode corresponding to the determined degree of the colour weakness as the at least one colour weakness mode of the colour weakness mode menu corresponding to the at least one determination mode menu if the at least one determination mode menu is selected.

19. A computer program which, when executed by a processor, causes the method steps of any one of the preceding claims to be performed.

20. A display apparatus comprising:
means for providing a user of a display with an interactive test to determine whether the user has defective colour vision; and
means for automatically adjusting output characteristics of the display in accordance with the determination.

21. A display apparatus according to claim 20, further comprising a display to display first and second graphic user interfaces, wherein the providing and adjusting means comprise:
a controller to determine whether a user has a colour weakness based on the displayed first graphic user interface, to determine a degree of the colour weakness based on the displayed second graphic user interface when it is determined that the user has the colour weakness, and to adjust display characteristics of the display according to the determined degree of the colour weakness.

22. A display apparatus according to claim 21, wherein the first graphic user interface comprises:
a plurality of test images sequentially displayed by the screen; and
a test input window corresponding to each of the plurality of test images to input test signals corresponding to each of the plurality test images.

23. A display apparatus according to claim 22, wherein the controller compares the input test signals corresponding to the sequentially displayed test images and determines that the user has the colour weakness when a predetermined number of the test signals are not identical to the corresponding test images.

24. A display apparatus according to claim 21, 22 or 23, wherein the second graphic user interface comprises:
a plurality of gradation boxes of each of a plurality of colours; and
a gradation range block corresponding to each colour to arrange the plurality of gradation boxes of the respective colour therein.

25. A display apparatus according to claim 24, wherein the controller compares the arrangement of the gradation boxes of each colour in the respective gradation range block with predetermined gradation box arrangements of each colour to determine the degree of the colour weakness with respect to each colour.

26. A display apparatus according to any one of claims 21 to 25, further comprising:
a user input part to input information into the first and second graphic user interfaces displayed on the screen.

27. A display apparatus according to any one of claims 21 to 26, further comprising:
an image processor to convert an image signal into a signal which is displayable by the screen; wherein the controller is arranged to control the display to display a first graphic user interface to determine whether a user has colour weakness and a second graphic user interface having an information graphic user interface to display information regarding a process of determining a degree of the colour weakness and at least one determination graphic user interface to determine the degree of the colour weakness based on at least one gradation of brightness and saturation with respect to at least one colour, to determine whether the user has the colour weakness based on a first test signal which is input through the first graphic user interface, to determine the degree of the colour weakness based on a second test signal which is input through the second graphic user interface, and to set a colour weakness mode corresponding to the determined degree of colour weakness.

28. A display apparatus according to claim 27, wherein the controller comprises:
a first controller which is provided in an external device to apply the image signal to the image processor, to apply graphic user interface image signals corresponding to the first graphic user interface and the second graphic user interface to the image processor, and to determine whether the user has the colour weakness and the degree of the colour weakness according to the first test signal and the second test signal; and
a second controller to control the image processor to change colour display characteristics of the image displayed on the display according to a control signal from the first controller corresponding to the determined degree of the colour weakness.

29. A display apparatus according to claim 28, wherein the first and second controllers communicate with each other through a DDC communication line.

30. A display apparatus according to claim 27, 28 or 29, wherein the controller controls the display to display a colour weakness set graphic user interface which includes a reset menu, a colour weakness test menu, and a colour weakness mode menu to select at least one colour weakness mode, and to display the first graphic user interface when the colour weakness test menu is selected.

31. A display apparatus according to any one of claims 27 to 30, wherein the controller controls the image processor to set colour display characteristics of the image displayed on the display to a predetermined normal mode when the reset menu is selected, and controls the image processor to set the colour display characteristics of the image displayed on the display to correspond to the selected at least one colour weakness mode when the at least one colour weakness mode of the colour weakness mode menu is selected.

32. A display apparatus according to any one of claims 27 to 31, wherein the colour weakness set graphic user interface further comprises a red control drag bar, a green control drag bar and a blue control drag bar, and
the controller activates the red control drag bar, the green control drag bar, and the blue control drag bar to be in a controllable state when one of the at least one colour weakness mode of the colour weakness menu is selected, and controls the image processor to adjust colour display characteristics of the image displayed on the display corresponding to the control of at least one of the red control drag bar, the green control drag bar and the blue control drag bar.

33. A display apparatus according to any one of claims 27 to 32, wherein the colour weakness set graphic user interface further comprises an initialization menu, and the controller initializes a preset colour weakness mode according to selection of the initialization menu if the initialization menu is selected.

34. A display apparatus according to any one of claims 27 to 33, wherein the first graphic user interface includes at least two colour weakness test images sequentially displayed on the screen, and the controller determines that the user has the colour weakness if the first test signal input corresponding to the sequentially displayed colour weakness test images is not identical with the colour weakness test images a predetermined number of times.

35. A display apparatus according to any one of claims 27 to 34, wherein the controller controls the display to display an information display window to display information that a user has a normal colour vision if the first test signal is identical with the colour weakness test images more than the predetermined number of times.

36. A display apparatus according to any one of claims 27 to 35, wherein the controller controls the display to display a mode set window which is provided with at least one determination mode menu corresponding to the at least one colour weakness mode of the colour weakness mode menu of the colour weakness set graphic user interface when the degree of the colour weakness is determined through the determination graphic user interface, and sets the colour weakness mode corresponding to the determined degree of the colour weakness as the at least one colour weakness mode of the colour weakness mode menu corresponding to the at least one determination mode menu if the at least one determination mode menu is selected.
